# EUROPEAN PATENT SPECIFICATION

(11) **EP 2 911 644 B1**
(45) Date of publication and mention of the grant of the patent: **07.08.2019**
(21) Application number: 13789376.4
(22) Date of filing: 25.10.2013
(51) Int. Cl.: A61K 8/11, A61K 8/19, B01J 13/02, A61K 33/24, A61K 9/16, A61K 8/24, A61K 8/25, A61Q 17/00, A61K 8/22, C11D 17/00, D06L 4/10, D06L 4/13, A61K 9/00

(54) **MULTI-COMPONENT ENCAPSULATED REACTIVE FORMULATIONS**
MEHRKOMPONENTIGE VERKAPSELTE REAKTIVE FORMULIERUNGEN
FORMULATIONS RÉACTIVES ENCAPSULÉES À PLUSIEURS CONSTITUANTS

(30) Priority: 26.10.2012 US 201261719158 P
(43) Date of publication of application: 02.09.2015
(73) Proprietor: Deb IP Limited, Denby, Derbyshire DE5 8JZ (GB)
(72) Inventor: HINES, John David, Audlem Cheshire CW3 0BA (GB); BINKS, Bernard Paul, Walkington Yorkshire HU17 8XX (GB); GRASCHA, Pierre Bruno, F-51350 Cormontreuil (FR)
(74) Representative: Elsy, David
(86) International application number: PCT/GB2013/052800
(87) International publication number: WO 2014/064464

(56) References cited:
- EP-A1- 0 510 761
- EP-A1- 1 320 412
- WO-A1-2008/072122
- WO-A1-2011/076518
- US-A1- 2009 148 342
- US-A1- 2009 252 815

## Description

### FIELD OF THE INVENTION

The present invention provides products including a least two reactants encapsulated in microdroplets which in turn are encapsulated by a coating of hydrophobic colloidal particles. The two reactants remain separately encapsulated up until the desired time of release at which time the reactants are released by mechanical action of the mixed colloidal particles such that when the two kinds of colloidal particles are mixed and mechanically treated, the reactants are released and whereupon the reactants react to form a desired product.

### BACKGROUND OF THE INVENTION

In many fields of application including but not limited to laundry or hard surface cleaning, personal hygiene, cosmetics, hair care, oral care, paints and finishes and animal nutrition, it is often highly desirable to deliver reactive ingredients at the point of need in their active state, such that beneficial reactions may take place when and where desired.

Non-limiting examples of such reactions may be bleaching of stains, sanitizing of hands, or formation of hard-set surface finishes. In all cases, a common difficulty to be overcome is the protection of the reactive ingredients up to the point of need, then the controlled release or controlled reaction of the same when desired.

A number of methods have been taught to overcome these issues including the use of dual-component packaging containers (for example as disclosed in US 3,685, 645 or US 5,881,869 to separate reacting materials prior to use, and the employment of a wide variety of encapsulating techniques including the use of encapsulating gels, polymers, waxes and films (see for example teaching disclosed in US 5,258,132 or US 6,248,364 or US 7,491,687).

While initially effective, a significant drawback of the dual-component packaging approach is that it is expensive to manufacture, cumbersome to operate and difficult to maintain in optimum state; a very common drawback being the consumption of one part of the reacting formulation at a faster rate than the other, leaving an ineffective residual amount at the end of use.

The most significant drawback of the available encapsulating approaches is that while often effective in preventing degradation of the reacting ingredients during storage and before use, they are generally less effective at providing a sufficient release of the ingredients at the point of need. While this may not present a significant problem in some applications where steady release over a period of time is desired, for example fragrance release from fabric conditioning products as disclosed US 4,446,032 or US 7,491,687, it is a drawback in a range of applications where rapid release and reaction is required, for example bleaching fabrics or hard surfaces, treatment of hair or teeth, sanitization of hands, curing of paints and finishes and provision of nutrients.

Furthermore, it is often the case that in order to overcome the above described problems, product formulators have taken recourse to using either less effective, less safe or less desirable ingredients which present fewer challenges in stability or reactivity. One such non-limiting example of this approach is in the field of hand hygiene where typically either chemical based biocides such as triclosan or chlorohexidine gluconate (CHG) are employed, or alcohol based sanitizers are used.

While stable in storage, a drawback of both is that they are typically only available in liquid based form and presented either as a liquid, a gel or foam. This requires the storage and logistical movement of significant quantities of liquid, which is cumbersome, expensive and potentially hazardous.

A further significant drawback of the chemical-based products is that all of the known and available bactericides are potentially hazardous to humans and are persistent on the skin.

A further drawback of the alcohol-based products available is the risk of fire due to the inherent flammability of the substances used.

Hydrogen Peroxide is well known in the art as a safe and highly effective disinfectant and sanitizer, however its use in hand hygiene has been restricted in the past due to a lack of effective means to overcome storage stability and rapid release challenges associated with this ingredient.

The concept of "dry water" has been well described in the art including for example publications such as "Binks, B.P.; Murakami, M; "Phase inversion of Particle-stabilized Materials from Foams to Dry Water", Nature Materials, Volume 5, November 2006" or disclosures such as U.S. Patent Publication No. 20030161855 or the earlier U.S. Patent No. 5,122,518. Dry water is formed when aqueous droplets are finely dispersed and stabilized by a hydrophobic powder material such as hydrophobized fumed silica or finely dispersed metal oxides. The appearance of "dry water" is as a fine, flowable powder which can be storage stable when handled correctly and within which the aqueous medium is maintained in an isolated state.

The idea of using "dry water" to carry effective ingredients including a range of biocides has been taught within the art such as U.S. Patent No. 5,122,518, however the compositions taught within are generally unstable over long periods of time. Further "dry water" mixtures including hydrogen peroxide are taught by U.S. Patent Publication No. 2009/0252815 wherein a range of hydrophobized silicas are proposed as the "protecting" phase and the resulting "dry water" powders are subsequently formulated within a range of cream formulations. Controlled, steady "time delayed" release of the hydrogen peroxide is well described by this teaching.

In both of the above and all other examples within the art however, no effective means to ensure rapid and effective release of the reacting ingredients are provided, such that while effectively "protected" during storage, no guarantee of rapid effectiveness at the point of need can be provided. Within U.S. Patent Publication No. 2009/0252815 for example, applications such as skin brightening or acne treatment are proposed wherein such "time delayed" release may be preferable, however this behavior would be wholly unsuitable and ineffective within the context of other applications such as skin sanitization or hard surface cleaning wherein a rapid "burst" of reaction is required.

U.S. Patent Publication No.2003/0157188 provides a bactericide useful as a shelf life extender for produce and other food products. The bactericide is an aqueous solution formed from water, copper sulfate pentahydrate and a reagent, which can either be an acid or hydrogen peroxide. The bactericide is applied as a coating on the food product and is especially useful in extending the shelf life of fruits and vegetables. Other applications of the bactericide include treatment of drinking water, bacteria and algae control in pools and natural bodies of water, and as a disinfectant in cleansers. The bactericide may further comprise a concentration of phosphoric acid.

U.S. Patent Application Publication No. 2008/0041794 teaches methods of decontaminating water, catalysts therefor and methods of making catalysts for decontaminating water to neutralize contaminants including organic and non-organic contaminants, such as aromatic compounds and microorganisms, e.g. bacteria. A heterogeneous catalyst is formed by incubating a polymeric resin with a transition metal-salt solution, e.g. a CuSO₄ solution. The contaminated water is treated by immersing the resulting heterogeneous catalyst in the contaminated water with hydrogen peroxide. The use of the Fenton reaction for providing a bactericide is disclosed.

U.S. Patent No. 4,311,598 relates to processes and compositions for the disinfection of aqueous media and particularly bacteria-containing aqueous effluents, e.g. treated municipal sewage or effluents from paper or food-processing industries, employing hydrogen peroxide-containing compositions as an alternative to chlorine. Specifically, the disinfectant comprises a combination of hydrogen peroxide, a soluble copper salt such as copper sulphate and an autoxisable reducing agent such as ascorbic acid or sodium sulphite, which can be employed in dilute concentrations at pH from 6 to 9, preferably 6.5 to 8. Particularly preferred combinations of the components are of mole ratios 1:1 to 60:1 of hydrogen peroxide:copper: and 5:1 to 1:1.2 copper:reducing agent.

U.S. Patent No. 5,780,064 provides an aqueous germicidal composition and related method for the treatment or prevention of infectious diseases of the hoof in animals, comprising a copper salt (such as copper sulfate), a quaternary ammonium compound, and a peroxide is disclosed.

U.S. Patent No. 5,681,591 discloses a composition useful for disinfecting a contact lens comprising a substantially isotonic, aqueous liquid medium containing hydrogen peroxide in an amount effective to disinfect a contact lens contacted with the aqueous liquid medium, and a hydrogen peroxide reducing agent dissolved in the aqueous liquid medium in an amount effective to enhance the antimicrobial activity of the aqueous liquid medium. Preferably, the composition further includes transition metal ions in an amount effective to further enhance the antimicrobial activity of the aqueous liquid medium and is substantially free of peroxidase.

U.S. Patent Publication No. 2010/0015245 describes a method of inhibiting biofilms by combinations of antimicrobials, particularly with their synergistic activity against biofilms. The antimicrobials include combination of copper ion and quaternary ammonium compound or combination of copper ion and peroxide. The invention also include methods for inhibiting biofilm-induced microbial corrosion or fouling.

U.S. Patent Publication No. 2010/0074967 teaches disinfectant or sterilant compositions, which are human safe, e.g., food grade or food safe. In one embodiment, an aqueous disinfectant or sterilant composition can comprise an aqueous vehicle, including water, from 0.001 wt % to 50 wt % of a peracid, and from 0.001 wt % to 25 wt % of a peroxide. Additionally, from 0.001 ppm to 50,000 ppm by weight of a transition metal based on the aqueous vehicle content can also be present. The composition can be substantially free of aldehydes. Alternatively or additionally, the transition metal can be in the form of a colloidal transition metal.

U.S. Patent Publication No. 2009/0252815 discloses pulverulent mixtures comprising hydrogen peroxide and hydrophobized, pyrogenically prepared silicon dioxide powder, preferably with a methanol wettability of at least 40. The pulverulent mixtures exhibit good storage stability and can be used for the controlled release of hydrogen peroxide and/or oxygen. The invention also includes methods of making these pulverulent mixtures and methods of using the mixtures in detergents, cleaning compositions, topical medications, antimicrobials and other products.

Methods are taught for the preparation of the pulverulent mixtures in which hydrogen peroxide is present in the form of drops of an aqueous solution which are enclosed by hydrophobized silicon dioxide. Such pulverulent mixtures can be produced by the intensive mixing of an aqueous hydrogen peroxide solution with hydrophobized silicon dioxide. Any mixing unit which can deliver sufficient energy to ensure a rapid division of the liquid into small droplets, which are then immediately surrounded by hydrophobized silicon dioxide powder, is suitable for this purpose.

U.S. Patent No. 6,861,075 discloses a storage-stable biocidal aerated gel composition that comprises from 30 to 97% by weight of water, from 0.2 to 5% by weight of a gelling agent selected from xanthan gum, sodium alginate and neutralised carboxyvinyl polymer from 2 to 5% by weight of a fine particulate, hydrophobic silicone-treated silica having a surface area of from 80 to 300 m2/g and from 0.004 to 20% by weight. The composition is a biocide which is in the form of fine particles of an aqueous gel containing the water, gelling agent and the biocide, the surfaces of which are coated with a coating of the finely particulate hydrophobic silica. The biocidal aerated gel composition is recommended for use in controlling pests using one or more appropriate biocides in the composition.

U.S. Patent No. 4,867,988 provides a stable oxygen-containing dentifrice in powder, paste or liquid form which contains a carrier and evenly dispersed therethrough a multiplicity of microencapsulated droplets of an oxygen releasing agent such as hydrogen peroxide. The walls of the microcapsules are rupturable upon mechanical manipulation of the dentifrice as it occurs during the toothbrushing action for releasing the oxygen-containing agent. The microcapsules are, however, not formed from hydrophobic silica, and are provided as a single component formulation only.

U.S. Patent Publication No. 2006/0276366 teaches a two-part hard surface treatment composition which is formed by the admixture of two aqueous compositions, particularly (a) an aqueous alkaline composition comprising a bleach constituent, with (b) an aqueous acidic composition comprising a peroxide constituent, which compositions are kept separate, but which are admixed immediately prior to use or upon use to form a foamed hard surface treatment composition.

U.S. Patent Publication No. US2006/0257498 provides an antimicrobial system having an amine part and an oxygen part, where the two parts are mixed prior to use to form an improved antimicrobial composition.

U.S. Patent Publication No. US2010/0143496 discloses a two-part disinfectant system which can be used to disinfect surfaces. The system includes a first chamber containing a first solution and a second chamber containing a second solution. The first solution can include an alcohol, an organic carboxylic acid, and from 0.01 ppm, to 1,000 ppm by weight of a transition metal or alloy thereof based on the first solution weight content. The second solution can include hydrogen peroxide. The system further includes a dispenser through which the system is configured to mix and dispense the first solution and the second solution immediately before being dispensed. A peracid composition is formed upon mixing of the first and second solutions.

Publication WO 2008/072122 A1 discloses personal care products, wherein hydrogen peroxide and a second reactant may be encapsulated separately.

### SUMMARY OF THE INVENTION

Accordingly, it is an object of the present invention to provide a means of effectively encapsulating and storing a range of reaction reagents suitable for use in a wide variety of potential applications as illustrated through the non-limiting list given above. Furthermore, it is an object of this invention to provide with such encapsulating systems a means of rapid release and effective reaction of the reaction reagents at the point/time of use. Specifically this is achieved by the use of multi-component encapsulated powder systems wherein one reaction reagent is maintained in isolation from one or more other reaction reagents up to the point of need when these are brought together by mechanical action.

Thus, a system for storing and releasing complementary reaction reagents, comprising:
a first part comprising colloidal particles, the colloidal particles coating and encapsulating microdroplets, the microdroplets comprising an aqueous solution including a first reaction reagent to form a first encapsulate containing said first reaction reagent;
at least a second part comprising colloidal particles, the colloidal particles coating and encapsulating microdroplets, the microdroplets comprising an aqueous solution including at least a second reaction reagent to form a second encapsulate containing said at least a second reaction reagent, said first and said at least a second reaction reagent being selected such that upon being mixed the first and at least a second reaction reagent chemically react to form reaction product; and
wherein in use the first and at least second parts, being commingled together, are subjected to mechanical action causing breakage of the first and at least second encapsulates releasing and mixing the first and at least second reaction reagents to form said reaction product is described.

The first and second colloidal particles may be hydrophobized colloidal particles, which can be fumed silica.

The system can be formulated as a skin sanitization product, as claimed, wherein the first aqueous solution comprises a peroxide, a first acid and water, and wherein the second aqueous solution comprises a salt of a transition metal, a second acid and water; and
wherein in use said first and second parts are applied on a user's skin and upon being subjected to mechanical action said first and second encapsulates are broken, releasing and mixing said first aqueous solution with said second aqueous solution.

The first acid and said second acids can be independently selected from the following: phosphoric acid, phosphonic acid, citric acid, sulfonic acid, sulphuric acid, hydrochloric acid, itaconic acid, methanoic acid, alpha-hydroxy acid, maleic acid, gluconic acid, propanoic acid, butanoic acid, pentanoic acid, hexanoic acid, heptanoic acid, octanoic mono-acid, octanoic di-acid, octanoic tri-acid, beta-hydroxy-acid, sodium tripolyphosphate, ethylenediaminetetraacetic acid, diethylenetriaminepentaacetic acid, N-(hydroxyethyl)-ethylenediami-netriacetic acid, 2-hydroxyethyliminodiacetic acid, benzoic acid, salicylic acid, aminobenzoic acid.

The transition metal can be selected from the following: iron, copper, manganese, cobalt, vanadium, titanium, chromium, lead, aluminum, gold and silver.

The first acid can be present in the first part in a range from about 0.1% to about 10% by weight. The second acid can be present in the second part in a range from about 0.1% to about 10% by weight.

The peroxide can be hydrogen peroxide present in the first part in a range from about 0.1% to about 70% by weight.

The salt can be present in the second part in a range from about 0.1% to about 70% by weight.

The water can be present in the first part in a range from about 50% to about 99% by weight. The water can be present in the second part in a range from about 50% to about 99% by weight.

When present, fumed silica can be present, for example, within the first and second parts in an amount less than about 5% by weight.

In embodiments, the transition metal is present at substantially the same molar concentration in the first part as the peroxide is present in the second part.

The system described can be formulated as a bleach system in which the first aqueous solution comprises water and one or combination of percarboxylic acid and bleach activator, and wherein the second aqueous solution comprises a salt of a transition metal and water. The one or combination of percarboxylic acid and bleach activator can be present in the first part in a range from about 1% to about 50% by weight. The salt can be present in the second part in a range from about 0.001ppm to about 49% by weight. The system can further include a third part the comprises one or combination of surfactant, enzyme, salt, perfume, colourant, and dye transfer inhibition agent.

The invention is a skin sanitization product, comprising:
a) a first part comprising first hydrophobized colloidal particles, said first hydrophobized colloidal particles containing first microdroplets, said first microdroplets comprising a first aqueous solution of a peroxide, a first acid and water to form a first encapsulate containing a first reaction element;
b) at least a second part comprising second hydrophobized colloidal particles, said second hydrophobized colloidal particles containing second microdroplets, said second microdroplets comprising a second aqueous solution of a salt of a transition metal, a second acid and water to form a second encapsulate containing a second reaction element; and
wherein in use said first and second parts are mixed together on a user's skin to form a mixture and upon being subjected to mechanical action said first and second encapsulates are broken, releasing and mixing said first and second reaction elements.

In a particular embodiment, the skin sanitization product includes a first part which comprises, by weight:
about 84% water; about 1% phosphoric acid (75%); about 12% hydrogen peroxide 50%; and about 3% fumed silica; and
a second part which comprises, by weight:
about 92.6% water; about 0.4% phosphoric acid (75%); about 4.0% copper sulphate; and about 3.0% fumed silica.

The bleach product, comprises:
a) a first part comprising first hydrophobized colloidal particles, said first hydrophobized colloidal particles containing first microdroplets, said first microdroplets comprising a first aqueous solution of water and one or combination of percarboxylic acid and bleach activator, to form a first encapsulate containing a first reaction element;
b) at least a second part comprising second hydrophobized colloidal particles, said second hydrophobized colloidal particles containing second microdroplets, said second microdroplets comprising a second aqueous solution of a salt of a transition metal and water to form a second encapsulate containing a second reaction element; and
wherein in use said first and second parts are mixed together to form a mixture and upon being subjected to mechanical action said first and second encapsulates are broken, releasing and mixing said first and second reaction elements.

The first part of the bleach product may comprise, by weight:
about 93% water; about 4.0% sodium percarbonate; about 3%fumed silica; and the second part comprises, by weight:
about 96.98% water; about 0.02% Mn(Bcyclam)Cl₂; and about 3.0% fumed silica.

The bleach product can further comprise a third part, wherein the third part comprises, by weight, about 50.0% zeolite; about 25.0% linear alkylbenzene sulphonate; about 15.0% C12-15 EO5 alcohol ethoxylate; and about 10.0% sodium silicate.

Also provided is a method for producing the skin sanitizing product for storing and releasing complementary reaction reagents, comprising the steps:
a) dissolving at least one first reaction reagent in water to form a first aqueous solution;
b) mixing and agitating said first aqueous solution with hydrophobized colloidal particles to form first colloidal particles encapsulating droplets of said first aqueous solution to form a first encapsulate;
c) dissolving at least one second reaction reagent in water to form at least one second solution;
d) mixing and agitating said second solution with said hydrophobized colloidal particles to form at least second colloidal particles encapsulating droplets of said second solution to form a second encapsulate; and
e) mixing said first and second encapsulate without breaking said first and second encapsulates to form an inter-dispersed composition of said first and second encapsulates.
wherein said at least one first reaction agent comprises a peroxide, a first acid and water and wherein said at least one second reaction reagent comprises a salt of a transition metal, a second acid and water.

Steps b) and d) can be accomplished with one of a high-shear rotary mixer and a blender.

The hydrophobized colloidal particles can comprise fumed silica, which may be present within said first and second colloidal particles in an amount less than about 5% by weight. The hydrophobized colloidal particles comprise metal oxide powders.

The at least one first reaction reagent comprises a peroxide, a first acid and water, and the at least one second reaction reagent comprises a salt of a transition metal, a second acid and water. The transition metal in the salt can be selected from the group consisting of iron, copper, manganese, cobalt, vanadium, titanium, chromium, lead, aluminum, gold and silver.

The first acid can be dissolved in the first solution in a range from about 0.1% to about 10%. The second acid can be dissolved in said second solution in a range from about 0.1% to about 10%. The peroxide can be hydrogen peroxide dissolved in the first solution in a range from about 0.1% to about 70%, and the salt can be dissolved in the second solution in a range from about 0.1% to about 70%.

After step b), the water can be present in the first solution in a range from about 50% to about 99%. After step d), the water can be present in the second solution in a range from about 50% to about 99%.

The salt of a transition metal is preferably present at substantially the same molar concentration in the first colloidal particles as the peroxide is present in the second colloidal particles.

In embodiments, the at least one first reaction reagent comprises an aqueous solution of water and one or combination of percarboxylic acid and bleach activator, and the at least one second reaction reagent comprises an aqueous solution of a salt of a transition metal and water. The one or combination of percarboxylic acid and bleach activator can be dissolved in the first solution in a range from about 1% to about 50% by weight. The salt can be dissolved in the second solution in a range from about 0.001 ppm to about 49% by weight.

The method can further include the steps of:
mixing a third composition, comprising of one or combination of surfactant, enzyme, salt, perfume, colourant, and dye transfer inhibition agent; and
mixing said inter-dispersed composition with said third composition.

The system can be as described above in which the first and second parts are included within a suitable carrier formulation selected from the group consisting of a cream, gel and paste.

The invention includes a method for delivering a skin sanitizing product, the method comprising:
(i) applying the product to the skin of a user, the product comprising a mixture of:
   A) a first part comprising a first encapsulate comprising hydrophobized colloidal particles and microdroplets comprising an aqueous solution of a peroxide, a first acid and water; and
   B) at least a second part comprising a second encapsulate comprising hydrophobized colloidal particles and microdroplets comprising an aqueous solution of the salt of a transition metal, a second acid and water; and
(ii) subjecting the product to mechanical force by hand-rubbing the product on the user's skin with sufficient force to break open the first and second encapsulates to release and mix said components of the first and second parts with each other.

The hydrophobized colloidal particles of the first and second parts can comprise fumed silica.

The first acid and said second acid can each include, independently of the other, one or more of phosphoric acid, phosphonic acid, citric acid, sulfonic acid, sulphuric acid, hydrochloric acid, itaconic acid, methanoic acid, alpha-hydroxy acid, maleic acid, gluconic acid, propanoic acid, butanoic acid, pentanoic acid, hexanoic acid, heptanoic acid, octanoic mono-acid, octanoic di-acid, octanoic tri-acid, beta-hydroxy-acid, sodium tripolyphosphate, ethylenediaminetetraacetic acid, diethylenetriaminepentaacetic acid, N-(hydroxyethyl)-ethylenediami-netriacetic acid, 2-hydroxyethyliminodiacetic acid, benzoic acid, salicylic acid, and aminobenzoic acid.

Described is a method for cleansing a surface, the method comprising the steps:
(i) applying the product to the surface, the product comprising a mixture of:
   (I) a first encapsulate comprising hydrophobized colloidal particles encapsulating an aqueous solution of at least one first reaction reagent; and
   (II) a second encapsulate comprising hydrophobized colloidal particles encapsulating an aqueous solution of at least one second reaction reagent;
   wherein the first and second reagents chemically react when exposed to each other to form a cleansing agent;
(ii) subjecting the product to mechanical force by hand-rubbing the product on the surface with sufficient force to break open the first and second encapsulates to release and mix said first and second reaction reagents and thereby expose the surface to the cleansing agent.

An embodiment of the present invention provides a skin sanitization product comprising a mixture of two dry powder components, where each dry powder component is formed from a phase inversion process involving vigorous mixing. The dry powder is stabilized by colloidal fumed silica particles, which assist in generating stable "dry water" microdroplets. One dry powder component comprises encapsulates containing microdroplets comprising an aqueous solution of hydrogen peroxide, and the other dry powder component comprises encapsulates containing microdroplets comprising an aqueous solution of copper sulphate or another suitable source of a transition metal ion. When the hand sanitization product is agitated by rubbing together of ones hands, the encapsulates rupture and the two aqueous solutions undergo a Fenton reaction upon contact. The Fenton reaction is catalyzed to produce hydroxyl free-radicals that generate an effective bactericidal dose.

### DETAILED DESCRIPTION OF THE INVENTION

Generally speaking, described herein is a system for storing and delivering two or more reaction reagents stored in microdroplets coated and encapsulated by hydrophobic colloidal particles to be released at the point and time of use by mechanical agitation of the stored reaction reagents in the encapsulates which are commingled such that the mechanical action releases the reagents which then react to give a desired reaction product. As required, embodiments of the present invention are disclosed herein. However, the disclosed embodiments are merely exemplary, and it should be understood that the invention may be embodied in many various and alternative forms. Some features may be exaggerated or minimized to show details of particular elements while related elements may have been eliminated to prevent obscuring novel aspects.

Therefore, specific structural and functional details disclosed herein are not to be interpreted as limiting but merely as a basis for the claims and as a representative basis for teaching one skilled in the art to variously employ the present invention. For purposes of teaching and not limitation, the illustrated embodiments are directed to a system for storing and delivering two or more reaction reagents stored in encapsulated microdroplets to be released at the point and time of desired use.

As used herein, the phrase "dry water" means the technology of microencapsulating water or aqueous solutions using hydrophobized colloidal silica particles described in the art including for example publications such as "Binks, B.P.; Murakami, M; "Phase inversion of Particle-stabilized Materials from Foams to Dry Water", Nature Materials, Volume 5, November 2006" or disclosures such as U.S. Patent Publication No. 2003/0161855 or the earlier U.S. Patent No. 5,122,518. The particles adsorb at the air/aqueous interfaces formed during vigorous agitation such that the formed aqueous microdroplets are stabilised by the adsorbed layer in the form of a finely dispersed dry powder. A thin layer of the powder settles between the liquid and the surrounding air and allows the coated water to retain a spherical shape for the droplet.

As used herein, the phrase "hydrophobized colloidal particles" means particles of fumed silica or metal oxides characterized as those with a methanol wettability of at least 40 and a specific surface area of between 10 and 400 m²/g, preferably between 80 and 300 m²/g.

As used herein, the phrase "microdroplets" means the droplets of water or aqueous solution finely dispersed within and stabilised by the adsorbed coating of hydrophobized fumed silica or metal oxides within the "dry water" composition. No definition or restriction upon the size or polydispersity of these droplets is implied or should be inferred from the informal use of the term "micro".

As used herein, the terms, "comprises" and "comprising" are to be construed as being inclusive and open ended, and not exclusive. Specifically, when used in this specification including claims, the terms, "comprises" and "comprising" and variations thereof mean the specified features, steps or components are included. These terms are not to be interpreted to exclude the presence of other features, steps or components.

As used herein, the coordinating conjunction "and/or" is meant to be a selection between a logical disjunction and a logical conjunction of the adjacent words, phrases, or clauses. Specifically, the phrase "X and/or Y" is meant to be interpreted as "one or both of X and Y" wherein X and Y are any word, phrase, or clause. The present invention relates to shelf stable skin sanitization and bleach products and methods of their preparation and use. Each of the product components as well and methods of their preparation are described in detail below.

Broadly speaking, described is a system for storing and releasing complementary reaction reagents at a desired time. This system generally comprises a first part comprising first colloidal particles, the first colloidal particles encapsulating first microdroplets, the first microdroplets comprising a first aqueous solution including a first reaction reagent to form a first encapsulate containing said first reaction reagent; at least a second part comprising second colloidal particles, the second colloidal particles encapsulating second microdroplets, the second microdroplets comprising a second aqueous solution including at least a second reaction reagent to form a second encapsulate containing said at least a second reaction reagent; said first and said at least a second reaction reagent being selected such that upon being mixed the first and at least a second reaction reagent chemically react to form a reaction product; and wherein in use the first and at least second parts, being commingled together, are subjected to mechanical action causing breakage of the first and at least second encapsulates releasing and mixing the first and at least second reaction reagents to form said reaction product.

The colloidal particles are hydrophobized colloidal particles and the microdroplets are aqueous microdroplets, thus the system is referred to a "dry water" system. In this embodiment, the storage stable "dry water" powders containing a range of reacting ingredients within the dispersed aqueous phase are disclosed. Two or more "dry water" powders are independently produced, each containing within the dispersed aqueous phase one reagent of a reaction mixture such that these ingredients are stored safely and effectively without reacting with each other. It is further disclosed that the independently produced "dry water" mixtures may be co-mixed with each other in a way that does not promote the breakdown of the dispersions, such that what is produced is a single, fully inter-dispersed powder containing all reaction reagents but wherein such reaction reagents remain isolated from each other such that stability is maintained.

The fully inter-dispersed, storage stable "dry water" mixture may then optionally be included within a suitable carrier formulation such as a cream, gel or paste according to the requirements of a given application. At the point of use, the fully inter-dispersed "dry water" powder is applied either solely or as part of a suitable formulation to the skin, fabric, hard surface or other point-of-use environment as required and through mechanical action such as rubbing or squeezing the inter-dispersed "dry water" powder is forced to break up allowing for the rapid and effective reaction of the stored reactive ingredients at the point of use. Each element of the invention is described in detail below. The colloidal particles may include, but are not restricted to, fumed silica.

The water used in the aqueous solutions may be purified to have a low electrical conductivity, for example less than about 50 microsiemens. This low conductivity promotes a longer shelf life for the encapsulated aqueous solutions. Methods for purifying water are well known in the art and may, for example, include reverse osmosis. It will be readily appreciated, however, that this discussion of water is purely didactic and should not limit the invention in any manner.

The present disclosure has application to many technical areas and is not restricted to any one in particular. For example, the present system is applicable to such areas including, but not limited to, laundry or hard surface cleaning, personal hygiene, pre-op skin disinfection, skin sanitization, surface disinfection, anti-dandruff treatment, cosmetics, hair care, oral care, paints and finishes and animal nutrition to mention just a few. The system finds utility in those areas where it is desirable to deliver reactive ingredients at the point of need in their active state, such that the chemical reactions of interest take place when and where desired.

Non-limiting examples of such reactions may be bleaching of stains, sanitizing of hands, nutrition of teeth, conditioning of hair, oxidation of free-radicals in the skin

The present invention will now be illustrated with the following exemplary, non-limiting Examples.

### EXAMPLES

The example compositions involve using hydrophobized colloidal particles and the microdroplets are aqueous microdroplets each containing different chemical reagents.

### "Dry water" stabilizing ingredient

The "dry water" mixtures described within the present invention comprise in general terms an aqueous phase within which are contained the reacting elements and a stabilizing ingredient which acts to stabilize the "dry water" dispersion and ensure isolation during storage of the reacting elements. Hydrophobized fumed silica (silicon dioxide) is proposed as a suitable stabilizing ingredient, for example, but not limited to, materials such as Aerosil R812, Aerosil R104, Aerosil R106 and Aerosil R812S all produced by Evonik Degussa GmBH of Essen, Germany.

Suitable hydrophobized fumed silica materials are characterized as those with a methanol wettability of at least 40 and a specific surface area of between 10 and 400 m²/g, preferably between 80 and 300 m²/g. Since the stabilizing ingredient is present only to provide suitable dispersion and storage stability, it is desirable to minimize the quantity in which it is present.

Accordingly, the stabilizing ingredient should be present within the final inter-dispersed "dry water" powder at levels of less than 10% by weight, preferably less than 5% by weight.

### Reacting ingredients

Within the disclosure a range of application areas are identified, each requiring the use of different suitable reactions in order to effect desired benefits. In all cases, at least two distinct reacting ingredients are identified (a "reaction mixture") and separated through manufacture and storage in order to ensure stability up to the point of use. It is within the scope of "reacting mixtures" that a broad range of chemistries and hence applications can be defined, thus we propose that non-limiting suitable and identified "reaction mixtures" include:

### Example 1: Hydrogen Peroxide + Transition Metal Ions (the Fenton Reaction) for Hand Sanitising Compositions

The invention provides a skin sanitization product, wherein the microdroplets encapsulated by an adsorbed coating of the first colloidal particles comprise an aqueous solution of a peroxide, acid and water, and wherein the microdroplets encapsulated by an adsorbed coating of the second colloidal particles comprise an aqueous solution of a salt of a transition metal, acid and water.

The Fenton reaction involves the use of transition metal ions, such as ions of iron, gold, silver, titanium, lead, aluminium, cobalt, chromium, vanadium, manganese, or copper though others are also used, to promote the breakdown on hydrogen peroxide to hydroxyl free-radicals. This reaction significantly increases the rapid effectiveness of hydrogen peroxide as a bleaching, oxidizing or biocidal active as it rapidly accelerates this critical breakdown step (free-radical production).

Within one embodiment of the invention, suitable compositions for effective production of the Fenton reaction at the point of use include the presence of hydrogen peroxide within the aqueous phase of one fraction of the stabilized "dry water" mixture at levels of between 1% and 70% by weight and preferably between 3% and 50% by weight.

Hydrogen peroxide is stabilized at low pH, therefore it is desirable to also include within the same dispersed aqueous phase a suitable acid buffer which may be selected from the list including but not limited to phosphoric acid, phosphonic acid, citric acid, sulfonic acid, sulphuric acid, hydrochloric acid, itaconic acid, methanoic acid, alpha-hydroxy acid, maleic acid, gluconic acid, propanoic acid, butanoic acid, pentanoic acid, hexanoic acid, heptanoic acid, octanoic mono-acid, octanoic di-acid, octanoic tri-acid, beta-hydroxy-acid, sodium tripolyphosphate, ethylenediaminetetraacetic acid, diethylenetriaminepentaacetic acid, N-(hydroxyethyl)-ethylenediami-netriacetic acid, 2-hydroxyethyliminodiacetic acid, benzoic acid, salicylic acid, and aminobenzoic acid. The amount of acid buffer included may vary but should be added such that pH of the said aqueous phase should be between 1 and 5, preferably 1 and 3.

The presence of a suitable transition metal ion within the aqueous phase of a second, separately stabilized fraction of the "dry water" mixture is required to produce the Fenton Reaction at the point of use. Suitable transition metal ions include but are not limited to iron, copper, manganese, cobalt, lead, aluminum, gold, titanium, chromium, vanadium and silver. Suitable forms for delivery of the transition metal ion into the aqueous phase include but are not limited to simple salts, ligand bound, dissolvable metal powder or dissolvable complex salts including glasses.

The Fenton reaction requires a stoichiometric mixture of transition metal ion and hydrogen peroxide. Accordingly it is desirable to include the selected transition metal ion in the same molar concentration as the inclusion of hydrogen peroxide in the final mixture. Suitable compositions are therefore produced by the inclusion of the selected transition metal ion between 0.1% and 70% by weight and preferably between 3% and 50% by weight.

### Example 2: Metal-ion catalysed bleach systems for Laundry or Dishwashing applications (not according to the invention)

It is well known in the art, as disclosed in for example US 6,306,812 that organic bleaches such as percarboxylic acids or other bleach activators may be made to perform considerably better in terms of stain bleaching on fabrics or hard surfaces in the presence of a suitable ligand-bound transition metal catalyst (of which several appropriate species are described in detail within US 6,306,812). A well known drawback as described in US 6,306,812 of this technology is the need to ensure that the catalytic reaction does not proceed on initial mixing but rather at the point of need.

Within one embodiment of the invention, suitable compositions for effective protection and subsequent delivery of transition metal catalysed bleaching of fabrics or hard surfaces at the point of use include the encapsulation of a suitable percarboxylic acid or bleach activator according to U.S. Patent No. 6,306,812 within the aqueous phase of one fraction of the stabilized "dry water" mixture at levels of between 0.001% and 99.99% by weight and preferably between 1% and 50% by weight.

The encapsulation of a suitable ligand-bound transition metal ion according to U.S. Patent No. 6,306,812 within the aqueous phase of a second, separately stabilized fraction of the "dry water" mixture is required to produce catalysis at the point of use. Suitable transition metal ions include but are not limited to ions of iron, copper, manganese, cobalt, chromium, vanadium, lead, aluminum, gold and silver. Suitable compositions are produced by the inclusion of the selected transition metal ion between 0.001 ppm and 49% by weight of the composition.

Preferably and optionally a third element of the composition should consist of suitable fabric or hard surface cleaning components selected from surfactants, enzymes, salts, perfumes, colourants, dye transfer inhibition agents and other suitable adjuncts. It is not necessary for these elements of the composition to be bound within either phase of the "dry water" encapsulates.

### Method of Manufacture

In all embodiments of the invention, at least two separate "dry water" compositions are first independently produced and then mixed to form the inter-dispersed, stabilized "dry water" mixture. In some embodiments, more than two reacting elements may be included, or more than one reaction mixture may be included such that the total number of independently produced "dry water" compositions may be unlimited in number. For the purposes of simple disclosure of the method of manufacture, an example including only two such independently produced compositions (**Part A** and **Part B**) is described, though for clarity it is noted that in fact any number of such compositions (**Part C, D, E,** etc) are possible. **Part A** is first produced independently by dissolving the first of the required reacting ingredients in water along with any required ancilliary ingredients. Using the example of the Fenton reaction composition given above, **Part A** would include the dissolution of hydrogen peroxide along with a suitable acid buffer ingredient to leave the composition at a suitable pH as defined above.

The aqueous solution of **Part A** is then mixed with a suitable amount of a selected stabilizing ingredient, such as hydrophobized fumed silica as defined above. Suitable mixing methods are high in energy and agitation and include the use of high-shear rotary mixers or blenders.

The resulting "dry water" powder represents **"Part A"** of the final composition and may be, for example, stored suitably in a plastic container until required later in the process.

**Part B** of the composition is then also produced independently by a method identical to **Part A.** In the example of the Fenton reaction given above, **"Part B"** would contain the transition metal ion reacting element, included at the same molar concentration as the hydrogen peroxide in **"Part A"** in order to produce a stoichiometric reaction. **Part B** is produced by first dissolving the required reacting elements and any ancillary ingredients in aqueous solution and then by mixing this solution with a suitably selected hydrophobized fumed silica stabilizing ingredient in a method identical to **"Part A".** The resulting "dry water" powder represents **"Part B"** of the final composition.

Once formed independently, **Parts A** and **B** of the composition are combined in equal parts with gentle mixing and avoiding breakage of the powder encapsulates to form the final inter-dispersed "dry water" composition according to the invention. The final inter-dispersed "dry water" composition is a dry powder in appearance that is stable and can be stored suitably, for example, in a plastic container or sachet.

As noted above, optionally additional **Parts C, D, E,** etc may be produced independently and inter-dispersed according to the specific requirements of a given application. Furthermore, the final inter-dispersed "dry water" composition may be optionally blended into a suitable cream, gel or paste to produce a formulation with appearance and properties suitable for the chosen application (e.g. application to face, skin, hair, teeth, fabric, hard surfaces, etc.).

### Method of Use

At the point of use, a suitable measure, according to the required application, of the final composition is applied to the skin (face, hands or other).

When the final composition is applied to the location of use, a mechanical action is required such as that provided by simple patting, squeezing, rubbing, spreading, brushing, polishing or scrubbing such that the inter-dispersed "dry water" encapsulates are broken, releasing and mixing the isolated reacting elements and causing the desired reaction(s) to take place rapidly and completely. It will be readily appreciated that the final composition may be subjected to such mechanical action in a different location than at the location of use. For example, encapsulates may be broken by the hands and applied to a surface for cleaning.

Using the example of the Fenton reaction composition described in Example 1, this could include without limitation the use of such composition as a hand sanitizing powder wherein a suitable amount of the final inter-dispersed composition is deposited in the hands and then, through vigorous rubbing, the "dry water" encapsulates are broken, releasing and mixing the hydrogen peroxide and selected transition metal ions and promoting the rapid and effective Fenton reaction, leading to effective disinfection of the hands.

### Example Compositions

1. Fenton reaction composition suitable for use as a hand sanitizing powder
a. **Part A** of the formulation consists of:

| Ingredient | Percentage (w/w %) |
|---|---|
| Water | 84 |
| Phosphoric Acid 75% | 1 |
| Hydrogen Peroxide 50% | 12 |
| Fumed Silica | 3 |

b. **Part B** of the formulation consists of:

| Ingredient | Percentage (w/w %) |
|---|---|
| Water | 92.6 |
| Phosphoric Acid 75% | 0.4 |
| Copper Sulphate | 4.0 |
| Fumed Silica | 3.0 |

2. Bleach catalyst composition suitable for use as a fabric or hard surface cleaner:
a. **Part A** of the formulation consists of

| Ingredient | Percentage (w/w %) |
|---|---|
| Water | 93.0 |
| Sodium Percarbonate | 4.0 |
| Fumed Silica | 3.0 |

b. **Part B** of the formulation consists of:

| Ingredient | Percentage (w/w %) |
|---|---|
| Water | 96.98 |
| Mn(BCyclam)Cl₂ | 0.02 |
| Fumed Silica | 3.0 |

c. **Part C** of the formulation (optionally encapsulated but not required) consists of:

| Ingredient | Percentage (w/w %) |
|---|---|
| Zeolite | 50.0 |
| Linear Alkylbenzene Sulphonate | 25.0 |
| C12-15 EO5 alcohol ethoxylate | 15.0 |
| Sodium silicate | 10.0 |

While a preferred form of this invention has been described above, it should be understood that the applicant does not intend to be limited to the particular details described above, but intends to be limited only to the scope of the invention as defined by the following claims.

Therefore the foregoing description of the preferred embodiments of the invention have been presented to illustrate the principles of the invention and not to limit the invention to the particular embodiment illustrated. It is intended that the scope of the invention be defined by all of the embodiments encompassed within the following claims.

## Claims

1. A skin sanitization product, comprising:
a) a first part comprising first hydrophobized colloidal particles, said first hydrophobized colloidal particles containing first microdroplets, said first microdroplets comprising a first aqueous solution of a peroxide, a first acid and water to form a first encapsulate containing a first reaction element; and
b) at least a second part comprising second hydrophobized colloidal particles, said second hydrophobized colloidal particles containing second microdroplets, said second microdroplets comprising a second aqueous solution of a salt of a transition metal, a second acid and water to form a second encapsulate containing a second reaction element; and
wherein in use said first and second parts are mixed together on a user's skin to form a mixture and upon being subjected to mechanical action said first and second encapsulates are broken, releasing and mixing said first and second reaction elements.

2. The skin sanitization product according to claim 1, wherein said first and second hydrophobized colloidal particles comprise fumed silica.

3. The skin sanitization product according to claim 1 or 2, wherein said first acid and said second acid are each selected, independently of the other, from the group consisting of phosphoric acid, phosphonic acid, citric acid, sulfonic acid, sulphuric acid, hydrochloric acid, itaconic acid, methanoic acid, alpha-hydroxy acid, maleic acid, gluconic acid, propanoic acid, butanoic acid, pentanoic acid, hexanoic acid, heptanoic acid, octanoic mono-acid, octanoic di-acid, octanoic tri-acid, beta-hydroxy- acid, sodium tripolyphosphate, ethylenediaminetetraacetic acid, diethylenetriaminepentaacetic acid, N-(hydroxyethyl)-ethylenediaminetriacetic acid, 2-hydroxyethyliminodiacetic acid, benzoic acid, salicylic acid, and aminobenzoic acid.

4. The skin sanitization product according to any one of claims 1 to 3, wherein said transition metal is selected from the group consisting of iron, copper, manganese, cobalt, vanadium, titanium, chromium, lead, aluminum, gold and silver.

5. The skin sanitization product according to any one of claims 1 to 4, wherein said first acid is present in said first part in a range from 0.1% to 10% by weight.

6. The skin sanitization product according to any one of claims 1 to 5, wherein said second acid is present in said second part in a range from 0.1% to 10% by weight.

7. The skin sanitization product according to any one of claims 1 to 6, wherein said peroxide is hydrogen peroxide present in said first part in a range from 0.1% to 70% by weight.

8. The skin sanitization product according to any one of claims 1 to 7, wherein said salt is present in said second part in a range from 0.1% to 70% by weight.

9. The skin sanitization product according to any one of claims 1 to 8, wherein said water is present in said first part in a range from 50% to 99% by weight

10. The skin sanitization product according to any one of claims 1 to 9, wherein said water is present in said second part in a range from 50% to 99% by weight.

11. The skin sanitization product according to any one of claims 1 to 10, wherein said hydrophobized colloidal particles are present within said first and second parts combined in an amount less than 5% by weight.

12. The skin sanitization product according to any one of claims 1 to 11, wherein said peroxide is present at the same molar concentration in said first part as said salt is present in said second part.

13. The skin sanitization product according to any one of claims 1 to 10, wherein said first part comprises, by weight:
84% water; 1% phosphoric acid (75%); 12% hydrogen peroxide 50%; and 3% fumed silica; and wherein said second part comprises, by weight:
92.6% water; 0.4% phosphoric acid (75%); 4.0% copper sulphate; and 3.0% fumed silica.

14. A method for producing the skin sanitizing product according to any of claims 1 to 13 for storing and releasing complementary reaction reagents, comprising the steps:
a) dissolving at least one first reaction reagent in water to form a first aqueous solution;
b) mixing and agitating said first aqueous solution with hydrophobized colloidal particles to form first colloidal particles encapsulating droplets of said first aqueous solution to form a first encapsulate;
c) dissolving at least one second reaction reagent in water to form at least one second solution;
d) mixing and agitating said second solution with hydrophobized colloidal particles to form at least second colloidal particles encapsulating droplets of said second solution to form a second encapsulate; and
e) mixing said first and second encapsulate without breaking said first and second encapsulates to form an inter-dispersed composition of said first and second encapsulates,
wherein said at least one first reaction reagent comprises a peroxide, a first acid and water, and wherein said at least one second reaction reagent comprises a salt of a transition metal, a second acid and water.

15. The method according to claim 14, wherein steps b) and d) are accomplished with one of a high-shear rotary mixer and a blender.

16. A method for delivering a skin sanitizing product according to any of claims 1 to 13, the method comprising:
(i) applying the product to the skin of a user, the product comprising a mixture of:
A) a first part comprising a first encapsulate comprising hydrophobized colloidal particles and microdroplets comprising an aqueous solution of a peroxide, a first acid and water; and
B) at least a second part comprising a second encapsulate comprising hydrophobized colloidal particles and microdroplets comprising an aqueous solution of the salt of a transition metal, a second acid and water; and
(ii) subjecting the product to mechanical force by hand-rubbing the product on the user's skin with sufficient force to break open the first and second encapsulates to release and mix said components of the first and second parts with each other.

## Patentansprüche

1. Hautdesinfektionsprodukt, umfassend:
a) einen ersten Teil, der erste hydrophobierte kolloidale Partikel umfasst, wobei die ersten hydrophobierten kolloidalen Partikel erste Mikrotropfen enthalten, wobei die ersten Mikrotropfen eine erste wässrige Lösung eines Peroxids, einer ersten Säure und Wasser umfassen, um eine erstes Verkapselungsmittel zu bilden, das ein erstes Reaktionselement enthält; und
b) mindestens einen zweiten Teil, der zweite hydrophobierte kolloidale Partikel umfasst, wobei die zweiten hydrophobierten kolloidalen Partikel zweite Mikrotropfen enthalten, wobei die zweiten Mikrotropfen eine zweite wässrige Lösung eines Salzes eines Übergangsmetalls, einer zweiten Säure und Wasser umfassen, um ein zweites Verkapselungsmittel zu bilden, das ein zweites Reaktionselement enthält; und
wobei bei Anwendung des Produkts der erste und der zweite Teil auf der Haut eines Benutzers zu einer Mischung miteinander vermischt werden, wobei unter mechanischer Krafteinwirkung das erste und das zweite Verkapselungsmittel aufgebrochen wird und es infolgedessen zur Freisetzung des ersten und des zweiten Reaktionselements und zu deren Vermischung miteinander kommt.

2. Hautdesinfektionsprodukt nach Anspruch 1, bei dem die ersten und die zweiten hydrophobierten kolloidalen Partikel pyrogene Kieselsäure umfassen.

3. Hautdesinfektionsprodukt nach Anspruch 1 oder 2, bei dem die erste Säure und die zweite Säure jeweils unabhängig voneinander ausgewählt sind aus der Gruppe bestehend aus Phosphorsäure, Phosphonsäure, Zitronensäure, Sulfonsäure, Schwefelsäure, Salzsäure, Itaconsäure, Methansäure, Alpha-Hydroxysäure, Maleinsäure, Gluconsäure, Propansäure, Butansäure, Pentansäure, Hexansäure, Heptansäure, Octanmonosäure, Octandisäure, Octantrisäure, Beta-Hydroxysäure, Natriumtripolyphosphat, Ethylendiamintetraessigsäure, Diethylentriaminpenta-essigsäure, N-(Hydroxyethyl)-ethylendiamintriessigsäure, 2-Hydroxyethyliminodiessigsäure, Benzoesäure, Salicylsäure und Aminobenzoesäure.

4. Hautdesinfektionsprodukt nach einem der Ansprüche 1 bis 3, bei dem das Übergangsmetall ausgewählt ist aus der Gruppe bestehend aus Eisen, Kupfer, Mangan, Kobalt, Vanadium, Titan, Chrom, Blei, Aluminium, Gold und Silber.

5. Hautdesinfektionsprodukt nach einem der Ansprüche 1 bis 4, bei dem die erste Säure in dem ersten Teil in einem Bereich von 0,1 bis 10 Gew.-% vorhanden ist

6. Hautdesinfektionsprodukt nach einem der Ansprüche 1 bis 5, bei dem die zweite Säure in dem zweiten Teil in einem Bereich von 0,1 bis 10 Gew.-% vorhanden ist.

7. Hautdesinfektionsprodukt nach einem der Ansprüche 1 bis 6, bei dem das Peroxid Wasserstoffperoxid ist, das in dem ersten Teil in einem Bereich von 0,1 bis 70 Gew.-% vorhanden ist.

8. Hautdesinfektionsprodukt nach einem der Ansprüche 1 bis 7, bei dem das Salz in dem zweiten Teil in einem Bereich von 0,1 bis 70 Gew.-% vorhanden ist.

9. Hautdesinfektionsprodukt nach einem der Ansprüche 1 bis 8, bei dem das Wasser in dem ersten Teil in einem Bereich von 50 bis 99 Gew.-% vorhanden ist.

10. Hautdesinfektionsprodukt nach einem der Ansprüche 1 bis 9, bei dem das Wasser in dem zweiten Teil in einem Bereich von 50 bis 99 Gew.-% vorhanden ist.

11. Hautdesinfektionsprodukt nach einem der Ansprüche 1 bis 10, bei dem die hydrophobierten kolloidalen Partikel in dem ersten und in dem zweiten Teil kombiniert in einer Menge von weniger als 5 Gew.-% vorhanden sind.

12. Hautdesinfektionsprodukt nach einem der Ansprüche 1 bis 11, bei dem das Peroxid in der gleichen molaren Konzentration in dem ersten Teil vorhanden ist wie das Salz in dem zweiten Teil.

13. Hautdesinfektionsprodukt nach einem der Ansprüche 1 bis 10, bei dem der erste Teil, jeweils auf das Gewicht bezogen, folgendes umfasst:
84% Wasser; 1% Phosphorsäure (75%); 12% Wasserstoffperoxid (50%); und 3% pyrogene Kieselsäure;
und wobei der zweite Teil, jeweils auf das Gewicht bezogen, folgendes umfasst:
92,6% Wasser; 0,4% Phosphorsäure (75%); 4,0% Kupfersulfat; und 3,0% pyrogene Kieselsäure.

14. Verfahren zur Herstellung des Hautdesinfektionsprodukts nach einem der Ansprüche 1 bis 13 zum Speichern und Abgeben komplementärer Reaktionsreagenzien, folgende Schritte umfassend:
a) Lösen mindestens eines ersten Reaktionsreagens in Wasser zur Bildung einer ersten wässrigen Lösung;
b) Mischen und Verrühren der ersten wässrigen Lösung mit hydrophobierten kolloidalen Partikeln zur Bildung erster kolloidaler Partikel, die Tröpfchen der ersten wässrigen Lösung verkapseln, wodurch ein erstes Verkapselungsmittel gebildet wird;
c) Lösen mindestens eines zweiten Reaktionsreagens in Wasser zur Bildung mindestens einer zweiten Lösung;
d) Mischen und Verrühren der zweiten Lösung mit hydrophobierten kolloidalen Partikeln zur Bildung mindestens zweiter kolloidaler Partikel, die Tröpfchen der zweiten Lösung verkapseln, wodurch ein zweites Verkapselungsmittel gebildet wird; und
e) Mischen des ersten und zweiten Verkapselungsmittel, ohne dabei das erste und das zweite Verkapselungsmittel aufzubrechen, um eine ineinander dispergierte Zusammensetzung aus dem ersten und dem zweiten Verkapselungsmittel zu bilden,
wobei das mindestens eine erste Reaktionsreagens ein Peroxid, eine erste Säure und Wasser umfasst, und wobei das mindestens eine zweite Reaktionsreagens ein Salz eines Übergangsmetalls, eine zweite Säure und Wasser umfasst.

15. Verfahren nach Anspruch 14, bei dem die Schritte b) und d) mittels entweder eines Rotationsmischgeräts mit hoher Scherwirkung oder eines Mixers durchgeführt werden.

16. Verfahren zur Verabreichung eines Hautdesinfektionsproduktes nach einem der Ansprüche 1 bis 13, wobei das Verfahren folgendes umfasst:
(i) Auftragen des Produkts auf die Haut eines Benutzers, wobei das Produkt eine Mischung aus folgendem umfasst:
A) einem ersten Teil, der ein erstes Verkapselungsmittel umfasst, welches hydrophobierte kolloidale Partikel und Mikrotropfen umfasst, wobei letztere eine wässrige Lösung eines Peroxids, einer ersten Säure und Wasser umfassen; und
B) mindestens einem zweiten Teil, der ein zweites Verkapselungsmittel umfasst, welches hydrophobierte kolloidale Partikel und Mikrotropfen umfasst, wobei letztere eine wässrige Lösung des Salzes eines Übergangsmetalls, einer zweiten Säure und Wasser umfassen; und
ii) Aussetzen des Produkts einer mechanischen Kraft, indem das Produkt mit ausreichender Kraft von Hand in die Haut des Benutzers eingerieben wird, um dadurch das erste und das zweite Verkapselungsmittel aufzubrechen, wodurch die Komponenten des ersten und zweiten Teils freigesetzt und miteinander vermischt werden.

## Revendications

1. Produit de nettoyage de la peau, comprenant :
a) une première partie comprenant de premières particules colloïdales rendues hydrophobes, lesdites premières particules colloïdales rendues hydrophobes contenant de premières micro gouttelettes, lesdites premières microgouttelettes comprenant une première solution aqueuse d'un peroxyde, d'un premier acide et d'eau pour former une première encapsulation contenant un premier élément de réaction ; et
b) au moins une seconde partie comprenant de secondes particules colloïdales rendues hydrophobes, lesdites secondes particules colloïdales rendues hydrophobes contenant de secondes micro gouttelettes, lesdites secondes micro gouttelettes comprenant une seconde solution aqueuse d'un sel d'un métal de transition, d'un second acide et d'eau pour former une seconde encapsulation contenant un second élément de réaction ; et
dans lequel lors de l'utilisation lesdites première et seconde parties sont mélangées ensemble sur une peau d'un utilisateur afin de former un mélange et lorsqu'elles sont soumises à une action mécanique lesdites première et seconde encapsulations sont cassées, libérant et mélangeant lesdits premier et second éléments de réaction.

2. Produit de nettoyage de la peau selon la revendication 1, dans lequel lesdites premières et secondes particules colloïdales rendues hydrophobes comprennent de la silice pyrogénée.

3. Produit de nettoyage de la peau selon la revendication 1 ou 2, dans lequel ledit premier acide et ledit second acide sont chacun sélectionnés, indépendamment de l'autre, à partir du groupe constitué par l'acide phosphorique, l'acide phosphonique, l'acide citrique, l'acide sulfonique, l'acide sulfurique, l'acide chlorhydrique, l'acide itaconique, l'acide méthanoïque, l'acide alpha-hydroxy, l'acide maléique, l'acide gluconique, l'acide propanoïque, l'acide butanoïque, l'acide pentanoïque, l'acide hexanoïque, l'acide heptanoïque, le monoacide octanoïque, le diacide octanoïque, le triacide octanoïque, l'acide bêta-hydroxy, le tripolyphosphate de sodium, l'acide éthylènediaminetétraacétique, l'acide diéthylènetriaminepentaacétique, l'acide N-(hydroxyéthyl)-éthylènediaminetriacétique, l'acide 2-hydroxyéthyliminodiacétique, l'acide benzoïque, l'acide salicylique, et l'acide aminobenzoïque.

4. Produit de nettoyage de la peau selon l'une quelconque des revendications 1 à 3, dans lequel ledit métal de transition est sélectionné à partir du groupe constitué par le fer, le cuivre, le manganèse, le cobalt, le vanadium, le titane, le chrome, le plomb, l'aluminium, l'or et l'argent.

5. Produit de nettoyage de la peau selon l'une quelconque des revendications 1 à 4, dans lequel ledit premier acide est présent dans ladite première partie dans une plage de 0,1 % à 10 % en poids.

6. Produit de nettoyage de la peau selon l'une quelconque des revendications 1 à 5, dans lequel ledit second acide est présent dans ladite seconde partie dans une plage de 0,1 % à 10 % en poids.

7. Produit de nettoyage de la peau selon l'une quelconque des revendications 1 à 6, dans lequel ledit peroxyde est le peroxyde d'hydrogène présent dans ladite première partie dans une plage de 0,1 % à 70 % en poids.

8. Produit de nettoyage de la peau selon l'une quelconque des revendications 1 à 7, dans lequel ledit sel est présent dans ladite seconde partie dans une plage de 0,1 % à 70 % en poids.

9. Produit de nettoyage de la peau selon l'une quelconque des revendications 1 à 8, dans lequel ladite eau est présente dans ladite première partie dans une plage de 50 % à 99 % en poids.

10. Produit de nettoyage de la peau selon l'une quelconque des revendications 1 à 9, dans lequel ladite eau est présente dans ladite seconde partie dans une plage de 50 % à 99 % en poids.

11. Produit de nettoyage de la peau selon l'une quelconque des revendications 1 à 10, dans lequel lesdites particules colloïdales rendues hydrophobes sont présentes dans lesdites première et seconde parties combinées dans une quantité inférieure à 5 % en poids.

12. Produit de nettoyage de la peau selon l'une quelconque des revendications 1 à 11, dans lequel ledit peroxyde est présent à la même concentration molaire dans ladite première partie que ledit sel est présent dans ladite seconde partie.

13. Produit de nettoyage de la peau selon l'une quelconque des revendications 1 à 10, dans lequel ladite première partie comprend, en poids :
84 % d'eau ; 1 % d'acide phosphorique (75 %) ; 12 % de peroxyde d'hydrogène 50 % ; et 3 % de silice pyrogénée ; et dans lequel ladite seconde partie comprend, en poids :
92,6 % d'eau ; 0,4 % d'acide phosphorique (75 %) ; 4,0 % de sulfate de cuivre ; et 3,0 % de silice pyrogénée.

14. Procédé de production du produit de nettoyage de la peau selon l'une quelconque des revendications 1 à 13 pour stocker et libérer des réactifs réactionnels complémentaires, comprenant les étapes de :
a) dissolution d'au moins un premier réactif réactionnel dans l'eau afin de former une première solution aqueuse ;
b) mélange et agitation de ladite première solution aqueuse avec des particules colloïdales rendues hydrophobes afin de former de premières particules colloïdales encapsulant des gouttelettes de ladite première solution aqueuse afin de former une première encapsulation ;
c) dissolution d'au moins un second réactif réactionnel dans l'eau afin de former au moins une seconde solution ;
d) mélange et agitation de ladite seconde solution aqueuse avec des particules colloïdales rendues hydrophobes afin de former au moins de secondes particules colloïdales encapsulant des gouttelettes de ladite seconde solution aqueuse afin de former une seconde encapsulation ; et
e) mélange de ladite première et de ladite seconde encapsulation sans casser lesdites première et seconde encapsulations afin de former une composition interdispersée desdites première et seconde encapsulations,
dans lequel ledit au moins un premier réactif réactionnel comprend un peroxyde, un premier acide et de l'eau, et dans lequel ledit au moins un second réactif réactionnel comprend un sel d'un métal de transition, un second acide et de l'eau.

15. Procédé selon la revendication 14, dans lequel les étapes b) et d) sont accomplies avec l'un d'un mixeur rotatif à haut cisaillement et d'un mélangeur.

16. Procédé de fourniture d'un produit de nettoyage de la peau selon l'une quelconque des revendications 1 à 13, le procédé comprenant :
(i) l'application du produit sur la peau d'un utilisateur, le produit comprenant un mélange de :
A) une première partie comprenant une première encapsulation comprenant des particules colloïdales rendues hydrophobes et des micro gouttelettes comprenant une solution aqueuse d'un peroxyde, d'un premier acide et d'eau ; et
B) au moins une seconde partie comprenant une seconde encapsulation comprenant des particules colloïdales rendues hydrophobes et des micro gouttelettes comprenant une solution aqueuse du sel d'un métal de transition, d'un second acide et d'eau ; et
(ii) la soumission du produit à une force mécanique par frottement à la main du produit sur la peau de l'utilisateur avec une force suffisante pour casser les première et seconde encapsulations afin de libérer et mélanger lesdits constituants des première et seconde parties les uns avec les autres.
